# EUROPEAN PATENT SPECIFICATION

(11) **EP 1 514 571 B1**
(45) Date of publication and mention of the grant of the patent: **18.01.2012**
(21) Application number: 04021479.3
(22) Date of filing: 09.09.2004
(51) Int. Cl.: A61M 25/02

(54) **Skin-penetrating medical object holder and blood pump system**
Halter für eine Hautpenetrationsvorrichtung und Blutpumpenanordnung
Support pour un dispositif médical de perforation et système de pompe à sang

(30) Priority: 12.09.2003 JP 2003322168
(43) Date of publication of application: 16.03.2005
(73) Proprietor: SUN MEDICAL TECHNOLOGY RESEARCH CORPORATION, Suwa-shi, Nagano (JP)
(72) Inventor: Yamazaki, Kenji, Koganei-shi Tokyo (JP); Oguchi, Kaori, Suwa-shi Nagano (JP); Ushiyama, Hiroyuki, Suwa-shi Nagano (JP)
(74) Representative: TBK

(56) References cited:
- US-A- 5 205 832
- US-A- 5 496 282
- US-B1- 6 436 074
- US-B1- 6 544 232

## Description

### BACKGROUND OF THE INVENTION

### 1. Field of the Invention

The present invention relates to a skin-penetrating medical object holder for holding a medical skin-penetrating object such as a tube, cable, or a catheter that passes through the skin of a living body, and to a blood pump system equipped with this skin-penetrating medical object holder. Such α holder is disclosed in document US 5496282.

### 2. Related Art

As examples, a medical skin-penetrating object, such as a tube, cable, or catheter that passes through the skin of a living body is used to connect the internal organ of the living body or the artificial organ and external medical equipment. By using such an object, an artificial heart can be used during and following heart surgery, or a variety of medical treatments, such as dialysis, peritoneal dialysis (hereinafter, "PD"), or the use of a drug delivery system, can be carried out. Infections due to the entry of bacteria into the living body at a part (hereinafter "tube entry part") where a tube or the like passes through (the skin of) the living body pose a significant clinical problem during such use and treatments. At the tube entry part, the bonding forces between the living body tissue near the skin and the surface of the tube are weak, so that external forces can easily detach the surface of the tube or the like from the living body tissue. Bacteria can enter the skin tissue at such parts, thereby causing infections. For this reason, it is necessary to take measures to make it difficult for external forces to act on the tube entry part, with this being an important step in the prevention of infections.

In order to solve the above problem, Yamazaki and Mori have developed a fixing construction as a medical assembly and has disclosed this construction in Japanese Laid-Open Patent Publication No. 2000-140125 (FIG 1), for example. FIG 15 is a cross-sectional view useful in explaining this fixing construction.

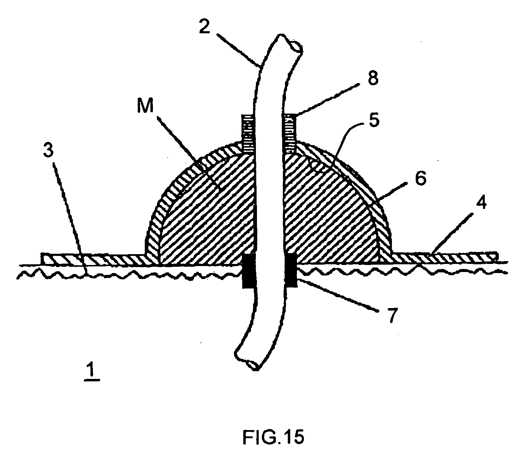

As shown in FIG 15, the fixing construction 1 is a medical assembly that prevents bacteria from entering a living body from a tube entry part where a tube 2 passes through the skin, and includes a sticking part 4, which is stuck onto the opening edge (the skin) 3 of the tube entry part, a covering 6 which holds and covers the tube entry part and forms an internal space 5, a tube holding part 7 for holding the tube 2 on the living body side, and a tube holding part 8 for holding the tube 2 that passes therethrough.

The fixing construction 1 makes it difficult for the tube 2 that passes through the skin of a living body to move, so that even if external forces are applied to the tube 2, detachment of the tube 2 from the living body tissue due to external forces is suppressed, which is effective in preventing infections. Additionally, when this fixing construction 1 is used, the internal space 5 of the covering 6 can be filled with medication M, so that infections can be suppressed even more effectively.

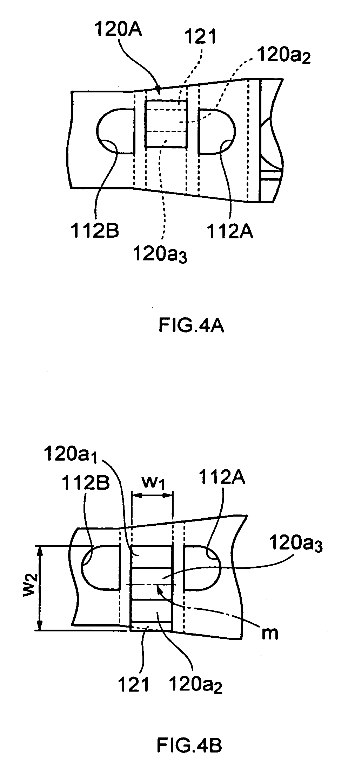

It should be noted that an abdominal belt for holding a tube or the like that passes through the skin of a living body has already been disclosed in the related art (see, for example, Japanese Registered Utility Model No. 3,066,243 (FIG 4)). Although Japanese Registered Utility Model No. 3,066,243 states that when not in use, a gastrostomy tube is stored inside a pocket of the abdominal belt to prevent detachment of the tube, there is no mention of a means for solving the problem of preventing infection from the outset by preventing the entry of bacteria into the living body.

However, if bacteria do enter the living body from the tube entry part of the skin and once an infection is caused at the periphery of the tube entry part of the skin, the infection progresses along the tube or the like deep into the living body tissue, which increases the risk of serious complications such as septicemia. For this reason, there have conventionally been demands for a medical assembly that reduces the load on a living body due to external forces applied to a tube or the like, can effectively suppress the detachment of the tube or the like and the living body tissue due to such external forces, and therefore can prevent the occurrence of infections from the outset.

### SUMMARY OF THE INVENTION

The present invention was conceived in response to the demands described above and it is an object of the present invention to provide a skin-penetrating medical object holder as further disclosed in claim 1 that can effectively suppress detachment of a medical skin-penetrating object and living body tissue due to external forces and therefore can prevent infections from occurring from the outset.
(1) To achieve the stated object, a skin-penetrating medical object holder according to the present invention includes: a belt-like holder main body that can be wound around a living body; and a penetrating object holding means that is disposed on the holder main body and holds a medical skin-penetrating object that passes through the skin of the living body.
   According to this skin-penetrating medical object holder, external forces applied to the medical skin-penetrating object can be received and absorbed by the penetrating object holding means and the holder main body that is wound around the living body.
   This means that it is possible to make movement of the medical skin-penetrating object with respect to the living body difficult, so that the load applied from the medical skin-penetrating object to the part where the object passes through the epidermis of the living body can be reduced. As a result, detachment of the medical skin-penetrating object and the living body tissue due to external forces can be effectively suppressed and therefore infections can be prevented from occurring from the outset.
   Here, the expression "penetrating" refers to the state where a medical skin-penetrating object such as a tube, cable, catheter or the like is introduced into a living body from outside the living body via the epidermis or a state where such object is guided out of the living body from within via the epidermis. The expression "medical skin-penetrating object" is not limited to tubes, cables and catheters and includes various kinds of objects that pass through the skin of a living body.
   For the skin-penetrating medical object holder according to the present invention, the penetrating object holding means should preferably be constructed so that in a state where the holder main body has been wound around the living body, the penetrating object holding means holds the medical skin-penetrating object in "a periphery of an entry point where the medical skin-penetrating object enters the skin of the living body".
   With the above construction, the length of a part to which the application of external forces is particularly undesirable (the gap between the medical skin-penetrating object and the penetrating object holding means) is shortened, so that it is possible to reduce the frequency with which external forces are applied to the part where the medical skin-penetrating object passes through the skin.
   By doing so, the load applied from the medical skin-penetrating object to the part where the object passes through the epidermis of the living body can be reduced. As a result, detachment of the medical skin-penetrating object and the living body tissue due to external forces can be effectively suppressed and therefore infections can be prevented from occurring from the outset.
   Here, the expression "a periphery of an entry point where the medical skin-penetrating object enters the skin of the living body" refers to a part where application of an external force is especially likely to cause detachment of the medical skin-penetrating object and the living body tissue, and more specifically is a part that is 20cm or less from the part where the medical skin-penetrating object passes through the skin of the living body. When an external force is applied to the medical skin-penetrating object in this part, detachment of the medical skin-penetrating object and the living body tissue is especially likely. From this viewpoint, it is more preferable to set "a periphery of an entry point where the medical skin-penetrating object enters the skin of the living body" as being 15cm or less from the part where the medical skin-penetrating object passes through the skin of the living body, and a setting of 10cm or less is even more preferable.
   For the skin-penetrating medical object holder according to the present invention, the penetrating object holding means should preferably be constructed so that in a state where the holder main body has been wound around the living body, the penetrating object holding means winds and holds the medical skin-penetrating object on the holder main body along a direction in which the medical skin-penetrating object is guided out of the living body.
   With the above construction, it is possible to make movement of the medical skin-penetrating object with respect to the living body difficult, so that the load applied from the medical skin-penetrating object to the part where the object passes through the epidermis of the living body can be further reduced. This means that detachment of the medical skin-penetrating object and the living body tissue due to external forces can be even more effectively suppressed and therefore infections can be prevented from occurring from the outset.
(2) For a skin-penetrating medical object holder of aspect (1) described above, the penetrating object holding means should preferably be constructed so as to guide, in a state where the holder main body has been wound around the living body, the medical skin-penetrating object in a length direction of the holder main body.
   With the above construction, even if an external force is applied to the medical skin-penetrating object due to movement of the living body or handling of the medical skin-penetrating object, the external force will be received and absorbed by the penetrating object holding means together with the holder main body. This means that it becomes even more difficult for the medical skin-penetrating object to move relative to the living body, so that the load applied from the medical skin-penetrating object to the part where the object passes through the epidermis of the living body can be further reduced. As a result, detachment of the medical skin-penetrating object and the living body tissue due to external forces can be even more effectively suppressed and therefore infections can be prevented from occurring from the outset.
   The skin-penetrating medical object holder according to the present invention should preferably be constructed so that when the holder main body is wound around a living body, the medical skin-penetrating object is guided around at least one quarter of one circumference in the length direction of the holder main body.
   With the above construction, even if an external force is applied to the medical skin-penetrating object due to movement of the living body or handling of the medical skin-penetrating object, it will be possible to absorb the external force more effectively. From this viewpoint, it is more preferable for the skin-penetrating medical object holder to be constructed so that the medical skin-penetrating object is guided around at least one third of one circumference in the length direction of the holder main body, with at least one half of one circumference being more preferable and at least two-thirds of one circumference being even more preferable.
(3) For a skin-penetrating medical object holder according to aspect (2) described above, the penetrating object holding means should preferably have a plurality of penetrating object holding parts that are disposed at predetermined intervals along the length direction of the holder main body.
   With the above construction, it is possible to easily and reliably guide the medical skin-penetrating object in the length direction of the holder main body. Since it is possible to disperse and absorb an external force that acts on the medical skin-penetrating object within the plurality of penetrating object holding parts, even if an external force is applied to the medical skin-penetrating object due to movement of the living body or handling of the medical skin-penetrating object, it will be possible to absorb the external force more effectively. The number of penetrating object holding parts can be set as appropriate in accordance with the size of the holder main body in the length direction.
(4) For a skin-penetrating medical object holder according to aspect (2) described above, the penetrating object holding means should preferably have a single penetrating object holding part that extends along the length direction of the holder main body.
   With the above construction, it is possible to easily and reliably guide the medical skin-penetrating object in the length direction of the holder main body. Since it is possible to effectively absorb an external force that acts on the medical skin-penetrating object within the penetrating object holding part that extends in the length direction of the holder main body, even if an external force is applied to the medical skin-penetrating object due to movement of the living body or handling of the medical skin-penetrating object, it will be possible to absorb the external force more effectively. The length of the penetrating object holding part in the length direction can be set as appropriate in accordance with the size of the holder main body in the length direction.
(5) For a skin-penetrating medical object holder according to any of aspect (1) to aspect (4) described above, the penetrating object holding means should preferably be constructed so as to guide, in a state where the holder main body has been wound around the living body, the medical skin-penetrating object in a length direction of the holder main body at substantially a same height as a height at an entry point where the medical skin-penetrating object passes through the skin of the living body.
   With the above construction, it is possible to make movement of the medical skin-penetrating object with respect to the living body difficult, so that the load applied from the medical skin-penetrating object to the part where the object passes through the epidermis of the living body can be further reduced. This means that detachment of the medical skin-penetrating object and the living body tissue due to external forces can be even more effectively suppressed and therefore infections can be prevented from occurring from the outset.
(6) For a skin-penetrating medical object holder according to any of aspect (1) to aspect (5) described above, the holder main body has a penetrating object passage hole through which the medical skin-penetrating object passes and a penetrating object guiding path that guides the medical skin-penetrating object in the penetrating object passage hole, and an edge member that opens and closes the penetrating object guiding path should preferably be attached to an edge of the penetrating object passage hole so as to be capable of separation and combining.
   With the above construction, the penetrating object guiding path is opened by separating the edge member from an edge of the penetrating object passage hole and the medical skin-penetrating object is introduced into the penetrating object passage hole from the penetrating object guiding path, so that the medical skin-penetrating object can be easily inserted into the penetrating object passage hole. It should be noted that after the medical skin-penetrating object has been inserted into the penetrating object passage hole, the edge member can be combined with the edge of the penetrating object passage hole.
(7) For a skin-penetrating medical object holder according to aspect (6) described above, the penetrating object passage hole should preferably be a hole for carrying out a medical treatment to an entry point where the medical skin-penetrating object passes through the skin of the living body and a periphery thereof.
   With the above construction, it is possible to carry out medical treatment, such as application of medication, to an entry point where the medical skin-penetrating object passes through the skin of the living body and a periphery thereof from the penetrating object passage hole.
(8) For a skin-penetrating medical object holder according to aspect (6) or aspect (7) described above, a wire-like member for reinforcing the skin-penetrating medical object holder should preferably be disposed at part of an edge of the penetrating object passage hole.
   With the above construction, the periphery of the penetrating object passage hole is reinforced by a wire-like member for reinforcing the holder. By doing so, it is possible to prevent the holder main body from losing its shape, so that the function of absorbing external forces can be maintained for a long period, and in turn infections can be prevented from occurring from the outset for a long period.
(9) For a skin-penetrating medical object holder according to aspect (8) described above, an end part of the wire-like member should preferably be composed of a flexible member.
   With the above construction, even if an external force is applied to the wire-like member of the holder main body towards the outside of the holder main body due to movement of the living body or handling of the medical skin-penetrating object, part of the external force will be absorbed by the flexible member. By doing so, it is possible to prevent the wire-like member in the holder main body from sticking out towards the outside of the holder main body. In this case, by curving the end part of the flexible member, the contact surface of the flexible member with respect to the holder main body can be widened and the load applied from the flexible member to the holder main body is reduced. For this reason, the wire-like member in the holder main body can be prevented from sticking out towards the outside of the holder main body more effectively.
(10) For a skin-penetrating medical object holder according to any of aspect (1) to aspect (9) described above, the penetrating object holding means should preferably include two attaching parts that can be detachably attached to one another and a penetrating object contacting part that is opened up and folded back via the attaching parts, and the penetrating object contacting part should preferably be constructed so that in a state where the two attaching parts are attached, the penetrating object contacting part is formed in a tube-like shape through which the medical skin-penetrating object is capable of passing.
   With the above construction, by placing the medical skin-penetrating object in contact with the penetrating object contacting part, folding back the penetrating object contacting part, and attaching the attaching parts together, it is possible to easily and reliably hold the medical skin-penetrating object.
(11) For a skin-penetrating medical object holder according to aspect (10) described above, the penetrating object contacting part should preferably be subjected to a treatment to stop the medical skin-penetrating object from slipping.
   With the above construction, the penetrating object contacting part effectively stops the medical skin-penetrating object from slipping. For this reason, it is possible to improve the holding of the medical skin-penetrating object by the penetrating object contacting part, so that movement of the medical skin-penetrating object with respect to the living body can be made even more difficult, and in turn the occurrence of infections can be prevented from the outset.
(12) For a skin-penetrating medical object holder according to any of aspect (1) to aspect (11) described above, an attaching part for attaching a supplementary holder, which is stuck onto the living body and holds the medical skin-penetrating object, should preferably be disposed on the holder main body.
   With the above construction, the medical skin-penetrating object can be held by the supplementary holder in addition to the penetrating object holding means. This means that the holding of the medical skin-penetrating object can be further improved, so that movement of the medical skin-penetrating object with respect to the living body can be made even more difficult, and in turn the occurrence of infections can be prevented from the outset.
   It should be noted that an opening for exposing one part or a front part of the supplementary holder to the outside can be preferably used as an attaching part for the supplementary holder. Here, a wound covering such as a specially manufactured holder, a gauze, or an adhesive plaster that is stuck onto the living body and holds the medical skin-penetrating object can be given as examples for the supplementary holder.
(13) For a skin-penetrating medical object holder according to aspect (12) described above, in addition to the attaching part, an extra attaching part should preferably be disposed on the holder main body a predetermined distance from the attaching part along the length direction of the holder main body.
   With the above construction, the attachment position of the supplementary holder can be changed, so that it is possible to prevent the supplementary holder from being stuck onto and used at the same position of the living body for a long period, so that inflammation and rashes can be prevented for the skin of the living body. In this case, even if inflammation has occurred, the supplementary holder can be moved to another attaching part.
(14) For a skin-penetrating medical object holder according to any of aspect (1) to aspect (13) described above, the holder tightening means for tightening the holder main body with respect to the living body should preferably be disposed on the holder main body, and the holder tightening means should preferably include: a holder locking part through which a first end of the holder main body passes and which locks the holder main body at a second end thereof; and a holder attaching part that is detachably attached to the first end of the holder main body that has been locked by the holder locking part and folded back.
   With the above construction, part of the holder main body is wound around the living body, one end of the holder main body is locked by the holder locking part, and this end is folded back and attached to the holder attaching part, so that the holder main body can be easily tightened to the living body. This means that it is possible to simply and reliably tighten the holder main body.
(15) For a skin-penetrating medical object holder according to any of aspect (1) to aspect (14) described above, the holder main body should preferably have an elastic part that is capable of stretching and contracting in at least the length direction.
   With the above construction, the holder main body can be wound around the living body even more favorably. In addition, since the holder main body has an elastic part, holders of the same length can be easily wound around living bodies of different physiques.
(16) For a skin-penetrating medical object holder according to any of aspect (1) to aspect (15) described above, a plurality of wire-like members, which are oriented in a width direction of the holder main body, for achieving tight contact between the living body and the skin-penetrating medical object holder should preferably be disposed in a row at predetermined distances in the length direction.
   With the above construction, it becomes possible to wind the holder main body around the living body more favorably, so that the holder main body can be favorably wound around living bodies of different physiques.
   When the medical skin-penetrating object is used together with a wound covering such as gauze, or an adhesive plaster, or a supplementary holder that assists in the holding of the medical skin-penetrating object, it is possible for the wire-like members to hold the wound covering and prevent it from becoming separated from the living body.
   It should be noted that a wire used in a woman's brassiere, a corrective corset, a waist nipper or the like, or a wire in the shape of a bar that has been wound in an irregular shape or a spiral can be used as the wire-like members. As the material of such members, a shape memory alloy, a metal such as SUS, or a resin such as PET may be used.
(17) For a skin-penetrating medical object holder according to any of aspect (1) to aspect (16) described above, the holder main body should preferably be formed of a woven material.
   With the above construction, the holder main body is wound around the living body in accordance with the shape of the wearing part of the living body. By doing so, the holder main body can be more favorably wound around the living body.
   In this specification, the woven material includes not only a woven material but also a knitted material.
(18) For a skin-penetrating medical object holder according to aspect (17) described above, the woven material should preferably be composed of fibers including a material that absorbs and radiates far-infrared radiation.
   With the above construction, far-infrared radiation from the living body can be well absorbed by the holder main body, so that a warm feel is obtained. Also, as a result, since moisture can be effectively dispersed from the holder main body, a dry feel is also obtained. This means that the occurrence of inflammation and the like of the skin of the living body can be effectively prevented, and a comfortable feel can be obtained.
   As a material that absorbs and radiates far-infrared radiation, ceramic particles that absorb and radiate far-infrared radiation can be favorably used. This is because such material can be kneaded into fibers or coated onto fibers easily.
   In the skin-penetrating medical object holder according to the present invention, aside from a textile composed of fibers that absorb and radiate far-infrared radiation, it is possible to favorably use a textile material composed of fibers (so-called "heat emitting fibers") that absorb moisture and give off heat as the woven material.
   With the above construction, the woven material is heated by absorbing humidity in the air and moisture from the living body, so that a warm feel is obtained. As a result, moisture is effectively dispersed from the holder main body and therefore a dry feel is obtained. This means that it is possible to effectively prevent the occurrence of inflammation in the skin of the living body and it is possible to obtain a comfortable feel. Acrylate fibers, polyester, and cellulose fibers can be given as examples of the material.
(19) For a skin-penetrating medical object holder according to aspect (17) or aspect (18) described above, the holder main body should preferably be subjected to a stitching process so that a holder thickness at outer edge parts thereof is thicker than a holder thickness at a central part thereof.
   With the above construction, the outer edge parts of the holder main body are reinforced compared to the central part. By doing so, it is possible to prevent the holder main body from losing its shape, so that the function of absorbing external forces can be maintained over a long period, and in turn it is possible to prevent the occurrence of infections from the outset over a long period.
(20) For a skin-penetrating medical object holder according to any of aspect (1) to aspect (19) described above, the medical skin-penetrating object should preferably be used to connect a blood pump implanted inside the living body and an external controller for driving and controlling the blood pump.
   With the above construction, it is possible to use a medical skin-penetrating object after an operation that implants a blood pump more safely and smoothly.
(21) For a skin-penetrating medical object holder according to any of aspect (1) to aspect (19) described above, the medical skin-penetrating object should preferably be used for circulating blood between the living body and a dialysis apparatus.
   With the above construction, it is possible to carry out dialysis treatment more safely and smoothly.
(22) For a skin-penetrating medical object holder according to any of aspect (1) to aspect (19) described above, the medical skin-penetrating object should preferably be used to carry out peritoneal dialysis.
   With the above construction, it is possible to carry out peritoneal dialysis treatment more safely and smoothly.
(23) For a skin-penetrating medical object holder according to any of aspect (1) to aspect (19) described above, the medical skin-penetrating object should preferably be used in a drug delivery system.
   With the above construction, it is possible to carry out treatment with a drug delivery system more safely and smoothly. That is, by carrying out treatment that directly administers medication to a diseased part by periodically providing a fixed amount of medication for a fixed period (a long period), it is possible to carry out such treatment more safely and smoothly.
   Here, the expression "drug delivery system" refers to a system where the administration of medication is optimized for treatment where medication is administered.
   It should be noted that aside from dialysis, PD, and a drug delivery system, the skin-penetrating medical object holder of the present invention can be favorably used for treatment that uses a PTCD tube, a T tube, or a gastrostomy tube, so that such treatment can be carried out more safely and smoothly.
(24) A blood pump system according to the present invention includes: a blood pump for being implanted inside a living body; an external controller for driving and controlling the blood pump; a medical skin-penetrating object for connecting the blood pump and the external controller; and a skin-penetrating medical object holder for holding the medical skin-penetrating object, wherein the skin-penetrating medical object holder is a skin-penetrating medical object holder according to any of aspect (I) to aspect (19).
   This means that the blood pump system of the present invention includes a skin-penetrating medical object holder that can prevent the occurrence of infections from the outset, and so is a blood pump system suited to treatment following an operation that implants a blood pump.

### BRIEF DESCRIPTION OF THE DRAWINGS

FIGS. 1A and 1B are views showing a skin-penetrating medical object holder according to an embodiment of the present invention;
FIG. 2 is a front elevation showing a state where the skin-penetrating medical object holder according to an embodiment of the present invention has been extended in a length direction;
FIGS. 3A and 3B are front elevations showing an enlargement of a part A in FIG. 1A;
FIGS. 4A and 4B are front elevations showing an enlargement of a part B in FIG. 1A;
FIGS. 5A and 5B are front elevations showing an enlargement of a part C in FIG. 1A;
FIG. 6 is a perspective view showing a state where the skin-penetrating medical object holder according to an embodiment of the present invention has been wound around a living body;
FIG. 7 is a front elevation showing a modification of the skin-penetrating medical object holder according to the embodiment of the present invention;
FIG. 8 is a perspective view showing a state where the skin-penetrating medical object holder shown in FIG. 7 is wound around a living body;
FIG. 9 is a horizontal cross-sectional view useful in explaining an example of winding the medical skin-penetrating object using a skin-penetrating medical object holder according to another embodiment of the present invention;
FIG. 10 is a perspective view useful in explaining a blood pump system that uses the skin-penetrating medical object holder according to an embodiment of the present invention;
FIG. 11 is a front elevation showing alternative usage 1 of the skin-penetrating medical object holder according to an embodiment of the present invention;
FIG. 12 is a perspective view showing a state where the skin-penetrating medical object holder shown in FIG. 11 is wound around a living body;
FIG. 13 is a front elevation showing alternative usage 2 of the skin-penetrating medical object holder according to an embodiment of the present invention;
FIG. 14 is a perspective view showing a state where the skin-penetrating medical object holder shown in FIG. 13 is wound around a living body; and
FIG. 15 is a cross-sectional view showing a conventional medical assembly.

### DESCRIPTION OF THE PREFERRED EMBODIMENTS

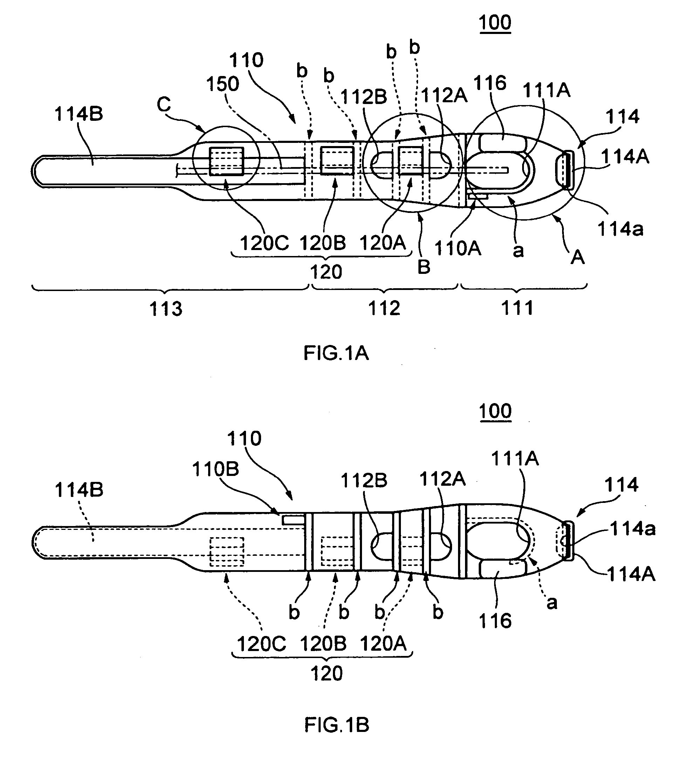

A skin-penetrating medical object holder and a blood pump system to which the present invention has been applied will be described below based on embodiments shown in the attached drawings. First, a skin-penetrating medical object holder will be described with reference to FIGS. 1A to 6.

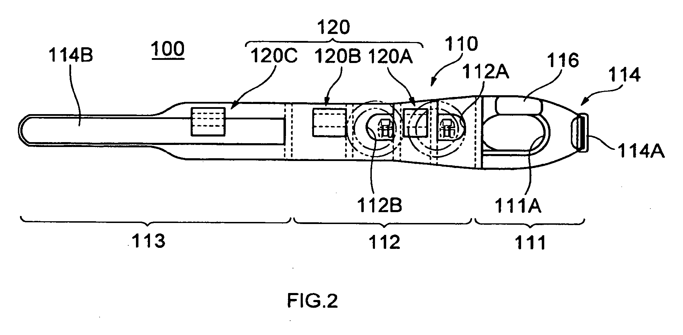

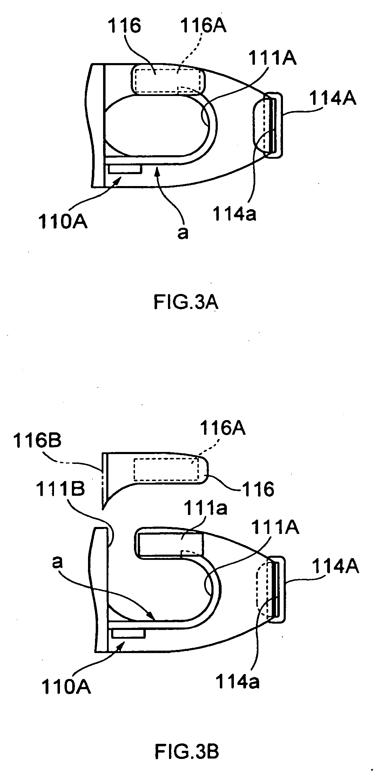

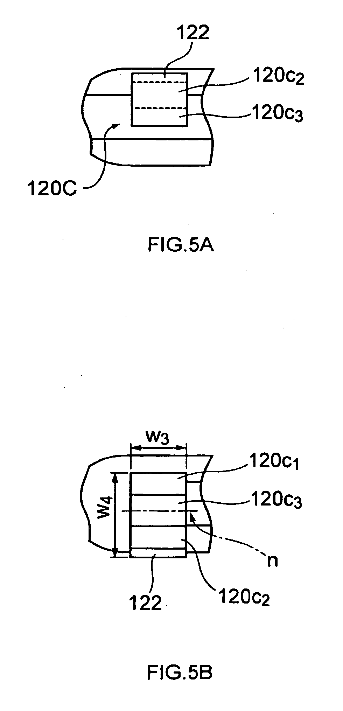

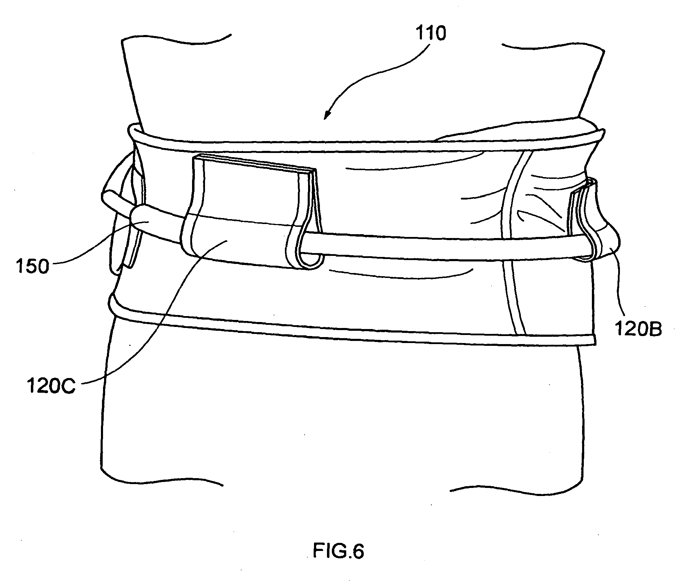

FIGS. 1A and 1B show a skin-penetrating medical object holder according to an embodiment of the present invention, with FIG. 1A is being a front elevation and FIG. 1B being a rear elevation. FIG. 2 is a front elevation showing a state where the skin-penetrating medical object holder according to an embodiment of the present invention has been opened out in the length direction. FIGS. 3A and 3B are front elevations showing an enlargement of the part A in FIG. 1A, with FIG. 3A being a front elevation showing a state where an edge member has been attached and FIG. 3B being a front elevation showing a state where the edge member has been separated. FIGS. 4A and 4B are front elevations showing an enlargement of the part B in FIG. 1A, with FIG. 4A being a front elevation showing a state where a penetrating object holding part has been folded back and FIG. 4B being a front elevation showing a state where the penetrating object holding part has been opened. FIGS. 5A and 5B are front elevations showing an enlargement of a penetrating object holding part in part C in FIG. 1A, with FIG. 5A being a front elevation showing a state where the penetrating object holding part has been folded back and FIG. 5B being a front elevation showing a state where the penetrating object holding part has been opened. FIG. 6 is a perspective view showing a state where the skin-penetrating medical object holder according to an embodiment of the present invention has been wound around a living body.

As shown in FIGS. 1A and 1B, a skin-penetrating medical object holder 100 according to an embodiment of the present invention includes a holder main body 110 and a penetrating object holding means 120, and is formed by an abdominal belt composed of a woven material (as one example, a textile material in the present embodiment). As one example of a textile material, a material composed of FIRBEST FIBER (a registered trademark of FIRBEST CO. LTD) including ceramic particles that absorb and radiate far-infrared radiation can favorably be used. By using such a material, in a wound state the skin-penetrating medical object holder 100 can absorb far-infrared radiation from the living body and moisture such as sweat can also be effectively dispersed from the skin-penetrating medical object holder 100. As a result, a warm, comfortable feel can be achieved during use, and inflammation and the like of the skin of the living body can be prevented. Also, since the skin-penetrating medical object holder 100 can be easily washed, the skin-penetrating medical object holder 100 can always be used in a clean state. It should be noted that reference numeral 110A in FIG. 1A designates a region for displaying a logo mark, while reference numeral 110B in FIG. 1B designates a region for displaying information such as a size.

The holder main body 110 includes three holder parts 111 to 113, and as shown in FIG. 6 is formed of a belt-like member that can be wound around a living body (such as a human body). This means that the holder main body 110 can be wound around the living body in accordance with the shape of the wearing part on the living body. For this reason, the winding force of the holder main body 110 on the living body can be increased.

The holder main body 110 is subjected to a stitching process to make the holder thickness at the outer edge parts thicker than the holder thickness at the center part. By doing so, the outer edge parts of the holder main body 110 can be reinforced relative to the center part of the holder main body 110. This means that the holder main body 110 can be prevented from losing its shape. A holder tightening means 114 is also disposed on the holder main body 110 to tighten the holder main body 110 on the living body.

The length L (the unstretched length) of the holder main body 110 in the length direction is set at 980mm, and the width W (maximum value) in the width direction is set at 140mm. It should be noted that the length of the holder main body 110 in the length direction can be set as appropriate for the dimensions of the wearing part on the living body.

The holder parts 111 to 113 are disposed in a row in the length direction and are set with respectively different dimensions (external forms) in the length and width directions. The holder part 111 is composed of an inelastic part in the shape of a curve so as to protrude in an up-down direction for the holder main body 110, and is disposed at one end (the right end in FIG. 1) of the holder main body 110. A locking member 114A is disposed at the front end (the free end) of the holder part 111 as a holder locking part that composes part of the holder tightening means 114. As shown in FIGS. 3A and 3B, this locking member 114A is formed of a substantially rectangular frame with a through hole 114a. The locking member 114A is attached to the holder part 111 by passing the end part of the holder part 111 through the through hole 114a, folding back the end part, and stitching the folded-back part to the non-folded back part of the holder part 111.

The holder part 111 includes a penetrating object passage hole 111A that allows a medical skin-penetrating object 150 to pass through and a penetrating object guiding path 111B for guiding the medical skin-penetrating object 150 within the penetrating object passage hole 111A, with an edge member 116 being stitched to an edge of the penetrating object passage hole 111A, the edge member 116 being attachable and detachable via fasteners 111a, 116A so as to open and close the penetrating object guiding path 111B. It should be noted that in FIG. 3B, reference numeral 116B designates a seam of the edge member 116. In this way, the insertion of the medical skin-penetrating object 150 into the penetrating object passage hole 111A is carried out by separating the edge member 116 from the edge of the penetrating object passage hole 111A to open the penetrating object guiding path 111B and guiding the medical skin-penetrating object 150 into the penetrating object passage hole 111A from the penetrating object guiding path 111B. It should be noted that after the medical skin-penetrating object 150 has been inserted into the penetrating object passage hole 111A, the edge member 116 is combined with the edge of the penetrating object passage hole 111A.

In the above, it is described that the edge member 116 is attachable and detachable via fasteners 111a, 116A, the present invention is not limited to this and an edge member may be attachable and detachable via buttons, strings, clasps such as hooks, magnets, buckles or the like.

The penetrating object passage hole 111A is constructed so as to function as a penetrating object passage hole for conducting a medical treatment. This means that a medical treatment, such as application of medication, can be carried out through the penetrating object passage hole 111A to the entry part of the skin, through which the medical skin-penetrating object 150 passes, and a periphery thereof. A medical assembly (not shown) that is stuck onto the living body can be disposed within the penetrating object passage hole 111A and a medical treatment can be carried out via this medical assembly. It should be noted that as one example of this type of medical assembly, the "fixing construction" disclosed by Yamazaki and Mori in Japanese Laid-Open Patent Publication No. 2000-140125 can be used.

At one part on the edge of the penetrating object passage hole 111A, a substantially J-shaped wire-like member (not shown) is embedded at a position shown by the arrows a in FIGS. 3A and 3B. The skin-penetrating medical object holder 100 (the holder main body 110) is also constructed so as to be reinforced. By doing so, the edge periphery of the penetrating object passage hole 111A is reinforced. This means that the holder main body 110 can be prevented from losing its shape. The front end of the wire-like member should preferably be composed of a flexible member. By doing so, even if an external force acts on the wire-like member inside the holder main body 110 towards the outside of the holder main body 110, part of such force will be absorbed by the flexible member. This means that within the holder main body 110, the wire-like member can be prevented from sticking out towards the outside of the holder main body 110. In this case, by carrying out a curved surface treatment of the flexible member, the contact area of the flexible member for the holder main body 110 is widened, so that the load applied on the holder main body 110 by the flexible member is reduced. This means that it is possible to prevent the wire-like member from sticking out towards the outside of the holder main body 110 more effectively.

The holder part 112 is composed of a linear-shaped elastic part that is elastic in at least the length direction and is disposed in a central position of the holder main body 110. By doing so, when the holder part 112 stretches as shown in FIG. 2, the stretching part is tightly wound around the living body. This means that the winding force of the holder main body 110 with respect to the living body can be increased. The holder main body 110 has an elastic part so that it is possible to wind a holder main body 110 of the same length around living bodies of different physiques. An opening 112A for exposing a supplementary holder (not shown) to the outside is provided at a penetrating object passage hole of the holder part 112, the supplementary holder sticking to the living body and holding the medical skin-penetrating object 150 in concert with the penetrating object holding means 120. In this way, the medical skin-penetrating object 150 is held by the penetrating object holding means 120 and the supplementary holder. By doing so, it is possible to more favorably hold the medical skin-penetrating object 150 on the holder main body 110.

At a substantially central part of the holder part 112 in the length direction, an extra opening 112B is provided in a line with and at a predetermined distance from the opening 112A in the holder length direction (on an opposite side of the holder part 111). By doing so, the medical skin-penetrating object 150 can be held not only by the penetrating object holding means 120 but also by the supplementary holder (not shown) that sticks to the living body and is exposed from the extra opening 112B. This means that it is possible to avoid having the supplementary holder stuck to the same part of the living body for a long period, so that inflammation and the like of the skin of the living body can be prevented.

It should be noted that the opening size of the openings 112A, 112B should preferably be set with consideration to the pressing of the supplementary holder onto the living body (by the edge parts of the openings 112A, 112B) when the holder main body 110 is wound and to the ease with which the supplementary holder can be stuck onto the living body. Here, if the sizes of the openings 112A, 112B are too small, although there will be a sufficient contact surface area for pressing the supplementary holder onto the living body, it will become difficult to stick the supplementary holder onto the living body. On the other hand, if the sizes of the openings 112A, 112B are too large, although it will become easier to stick the supplementary holder onto the living body, a sufficient contact surface area for pressing the supplementary holder onto the living body will not be achieved.

At the plurality of positions shown by the arrows b in FIGS. 1A and 1B, wire-like members, which are oriented in the width direction of the holder, for achieving tight contact between the living body and the holder are embedded. These wire-like members are formed by a plurality of loop members disposed in a row in the length direction of the holder with predetermined distances in between. By doing so, it is possible to wind the holder main body 110 around the living body with tight contact being achieved at a plurality of positions in the length direction due to these wire-like members. This means that it is possible to effectively increase the winding force of the holder main body 110 on the living body.

The holder part 113 is composed of two inelastic parts with respectively different widths, and is disposed at the other end of the holder main body 110. On the surface side of the holder part 113, a fastener 114B is disposed as a holder attaching part that in concert with the locking member 114A composes the holder tightening means 114. In this way, one end of the holder main body 110 (the holder part 113) that is locked by (passes through) the locking member 114A and is folded back is constructed so as to be attachable and detachable. By doing so, the holder main body 110 can be tightened on the living body by the holder tightening means 114 by winding one part (a middle part) of the holder main body 110 around the living body, passing the holder main body 110 (the holder part 113) through the through hole 114a to lock the holder main body 110 with the locking member 114A, and finally folding back the holder part 113 and attaching the fastener 114B. In this case, the holder main body 110 is tightened to the living body when the holder part 113 is attached to the fastener 114B. This means that it is possible to easily and reliably tighten the holder main body 110 using the holder tightening means 114.

On the other hand, as shown in FIGS. 1A and 1B, the penetrating object holding means 120 is composed of a penetrating object holding means that holds the medical skin-penetrating object 150, which is passed through the skin of the living body, on the outside of the living body, and is disposed on the holder main body 110 (the holder parts 112, 113). The penetrating object holding means 120 is constructed so as to guide the medical skin-penetrating object 150 along the length of the holder main body 110 in the wound state for the holder. The penetrating object holding means 120 includes a plurality of penetrating object holding parts 120A to 120C that are aligned at predetermined intervals along the length direction of the holder main body 110. By doing so, external forces that act on the medical skin-penetrating object 150 are dispersed and absorbed by the plurality of penetrating object holding parts 120A to 120C. In this case, the penetrating object holding parts 120A to 120C should preferably be disposed at positions so that the medical skin-penetrating object 150 is guided around at least one half circumference of the wearing part of the living body. By doing so, a state where the medical skin-penetrating object 150 is stably held on the living body is achieved. It should be noted that the number of penetrating object holding parts 120A to 120C can be set as appropriate in accordance with the size of the holder main body 110 in the length direction. It should be noted that although the case where there are three penetrating object holding parts is described in the present embodiment, the number can be set as appropriate in accordance with the length of the holder. The lengths of the penetrating object holding parts are also set as appropriate in accordance with the length of the holder.

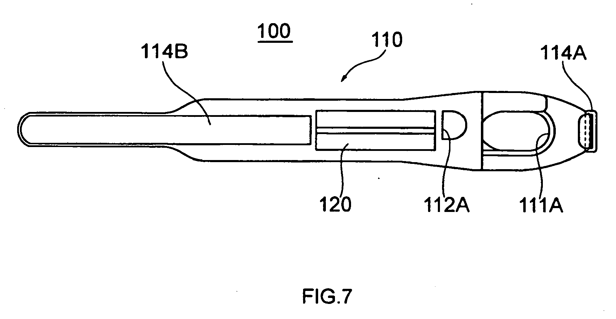

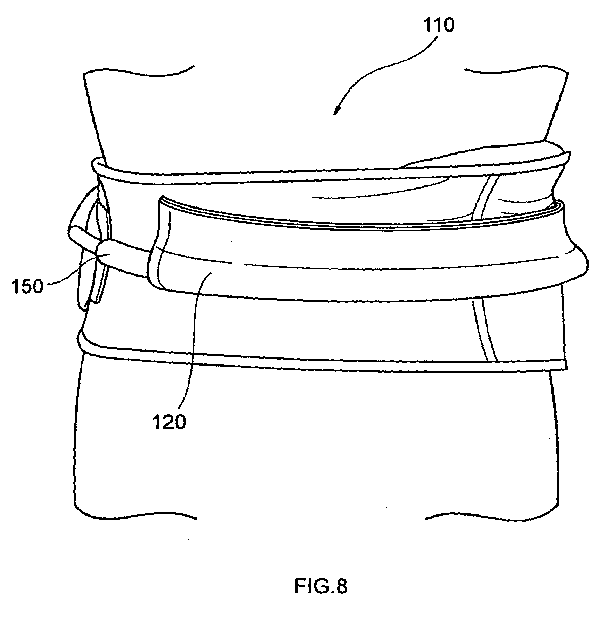

Here, as one example, when the penetrating object holding means 120 of the skin-penetrating medical object holder 100 is composed of a single penetrating object holding part as shown in FIG. 7, the length of the penetrating object holding part in the holder length direction should preferably be increased. To attach the skin-penetrating medical object holder 100 to the living body, the holder main body 110 is wound around the living body as shown in FIG. 8. By doing so, external forces that act on the medical skin-penetrating object 150 are absorbed by the entire penetrating object holding part (the penetrating object holding means 120) that extends in the length direction of the holder main body 110. In this case, the penetrating object holding part should preferably be disposed at positions so that the medical skin-penetrating object 150 is guided around at least one half circumference of the wearing part of the living body. By doing so, a state where the medical skin-penetrating object 150 is stably held on the living body is achieved. The length of the penetrating object holding part is also set as appropriate in accordance with the holder length.

The penetrating object holding parts 120A to 120C are disposed at substantially the same height as the height of the position where the medical skin-penetrating object 150 passes through the skin of the living body. By doing so, the penetrating object holding parts 120A to 120C can be wound around the wearing part of the living body at substantially the same height as the part at which the medical skin-penetrating object 150 passes through the skin of the living body. This means that the load applied from the medical skin-penetrating object 150 to the part where the object passes through the epidermis of the living body can be further reduced. By doing so, it is possible to effectively suppress detachment of the medical skin-penetrating object 150 and the living body tissue due to external forces, so that infections can be prevented from the outset even more effectively. As shown in FIGS. 4A and 4B, the penetrating object holding part 120A includes two attaching parts 120a₁, 120a₂ that can be detachably attached to each other and a penetrating object contacting part 120a₃ that can be opened up and folded back via both attaching parts 120a1, 120a2, and is disposed between the two openings 112A, 112B. The width w₁ of the penetrating object holding part 120A in the left-right direction is set at 45mm and the width w₂ in the up-down direction in an opened-up state for the penetrating object contacting part 120a₃ is set at 96mm.

One attaching part 120a₁ out of the attaching parts 120a₁, 120a₂ is attached to an upper end of a surface of the holder part 112, with a grasping tab 121 used during attachment and detachment being disposed on the other attaching part 120a₂. The penetrating object contacting part 120a₃ is composed so as to be formed in a tube-like form through which the medical skin-penetrating object 150 passes when both attaching parts 120a₁, 120a₂ are in the attached state. In this way, to have the medical skin-penetrating object 150 held by the penetrating object holding part 120A, the medical skin-penetrating object 150 is placed in contact with the penetrating object contacting part 120a₃ and the attaching parts 120a₁, 120a₂ are attached to one another by folding back the penetrating object contacting part 120a₃ with the imaginary line shown by the symbol m in FIG. 4B, for example, as the folding back line. In this case, when the attaching parts 120a₁, 120a₂ are attached to one another, the penetrating object contacting part 120a₃ is formed in a tube-like part, with the medical skin-penetrating object 150 being held by this tube-like part. By doing so, it is possible to simply and reliably have the medical skin-penetrating object 150 held by the penetrating object holding part 120A.

A surface treatment to prevent the medical skin-penetrating object 150 from slipping is carried out on the penetrating object contacting part 120a₃. By doing so, it is possible to stop the medical skin-penetrating object 150 slipping in the penetrating object contacting part 120a₃. This means that it is possible to improve the holding of the medical skin-penetrating object 150 on the holder main body 110 by the penetrating object holding part 120A.

The penetrating object holding parts 120B and 120C have substantially the same construction (with only the attachment positions differing), and therefore of these, only the penetrating object holding part 120C will be described as a representative example. As shown in FIGS. 5A and 5B, the penetrating object holding part 120C includes two attaching parts 120c₁, 120c₂ that can be detachably attached to each other and a penetrating object contacting part 120c₃ that is opened up and folded back via both attaching parts 120c₁, 120c₂. The width w₃ of the penetrating object holding part 120C in the left-right direction is set at 60mm and the width w₄ in the up-down direction in an opened-up state for the penetrating object contacting part 120c₃ is set at 96mm.

One attaching part 120c₁ out of the attaching parts 120c₁, 120c₂ is attached to an upper end edge of a surface of the holder part 113. A grasping tab 122 used during attachment and detachment is disposed on the other attaching part 120c₂. The penetrating object contacting part 120c₃ is composed so as to be formed in a tube-like form through which the medical skin-penetrating object 150 passes when both attaching parts 120c₁, 120c₂ are in the attached state. In this way, to have the medical skin-penetrating object 150 held by the penetrating object holding part 120C, the medical skin-penetrating object 150 is placed in contact with the penetrating object contacting part 120c₃ and the attaching parts 120c₁, 120c₂ are attached to one another by folding back the penetrating object contacting part 120c₃ with the imaginary line shown by the symbol n in FIG. 5, for example, as the folding back line. In this case, when the attaching parts 120c₁, 120c₂ are attached to one another, the penetrating object contacting part 120c₃ is formed in a tube-like part, with the medical skin-penetrating object 150 being held by this tube-like part. By doing so, it is possible to simply and reliably have the medical skin-penetrating object 150 held by the penetrating object holding part 120C.

A surface treatment to prevent the medical skin-penetrating object 150 from slipping is carried out on the penetrating object contacting part 120c₃. By doing so, it is possible to stop the medical skin-penetrating object 150 slipping in the penetrating object contacting part 120c₃. This means that it is possible to improve the holding of the medical skin-penetrating object 150 onto the holder main body 110 by the penetrating object holding part 120C. Here, a surface treatment, such as formation of a polyurethane coating on nylon tricot or an acryl coating on polyester tricot, or attachment of a silicon sheet or chamois can be given as examples of the treatment carried out for the penetrating object contacting part 120c₃.

It should be noted that the above penetrating object holding means preferably includes attaching parts composed of a fastener, for example.. In addition, the above penetrating object holding means may include attaching parts having buttons, strings, clasps such as hooks, magnets, buckles or the like. Further, the above penetrating object holding means may be composed of a clip in which the medical skin-penetrating object is caught, a partly cut ring through which medical skin-penetrating object is inserted, or the like. In short, the above penetrating object holding means should preferably be such means that the medical skin-penetrating object can be hold in any way.

With the above construction, the holder main body 110 of the skin-penetrating medical object holder 100 according to the present embodiment is wound around the living body and the medical skin-penetrating object 150 is held by the penetrating object holding means 120, so that even if an external force is applied to the medical skin-penetrating object 150 due to movement of the living body or handling of the medical skin-penetrating object 150, the external force will be received and absorbed by the penetrating object holding means 120 together with the holder main body 110 wound around the living body.

Accordingly, with the skin-penetrating medical object holder 100 according to the present embodiment, loads placed on the medical skin-penetrating object 150 can be reduced, so that it becomes difficult for the medical skin-penetrating object 150 to move relative to the living body. As a result, detachment of the medical skin-penetrating object 150 and the living body tissue due to external forces can be effectively suppressed, so that infections can be prevented from the outset.

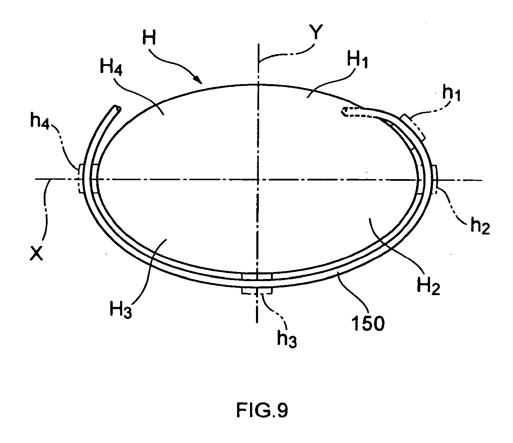

Here, as shown in FIG. 9 for example, in a case where four penetrating object holding parts h₁ to h₄ (the above embodiment has three) are disposed and the skin-penetrating medical object holder 100 is wound around the abdominal part of a human body H as an abdominal belt, a cross-section of the abdominal part is divided into four regions H₁ to H₄ (four substantially equal parts) by two perpendicular imaginary lines X and Y, and the skin-penetrating medical object holder 100 is wound so as to guide the medical skin-penetrating object 150 across positions (the epidermis) corresponding to these four regions H₁ to H₄, the medical skin-penetrating object 150 will be stably held on the human body H, so that detachment of the medical skin-penetrating object 150 and the living body tissue due to an external force is effectively prevented, which means that infections can be prevented even more effectively.

It should be noted that when the holder is in the wound state, out of the penetrating object holding parts h₁ to h₄, the holding of the medical skin-penetrating object 150 by the penetrating object holding part h₁ near the entry point is most important, with the holding of the medical skin-penetrating object 150 by the penetrating object holding parts h₂, h₃ where the curvature of the human body is greatest (where the curvature of the medical skin-penetrating object 150 is also large) being next in importance. Here, the greatest effect is achieved in the case where the medical skin-penetrating object 150 is held by a single penetrating object holding part or a plurality of penetrating object holding parts from the entry point of the medical skin-penetrating object 150 into the living body to the penetrating object holding part h₄, that is, around at least one half circumference of the living body.

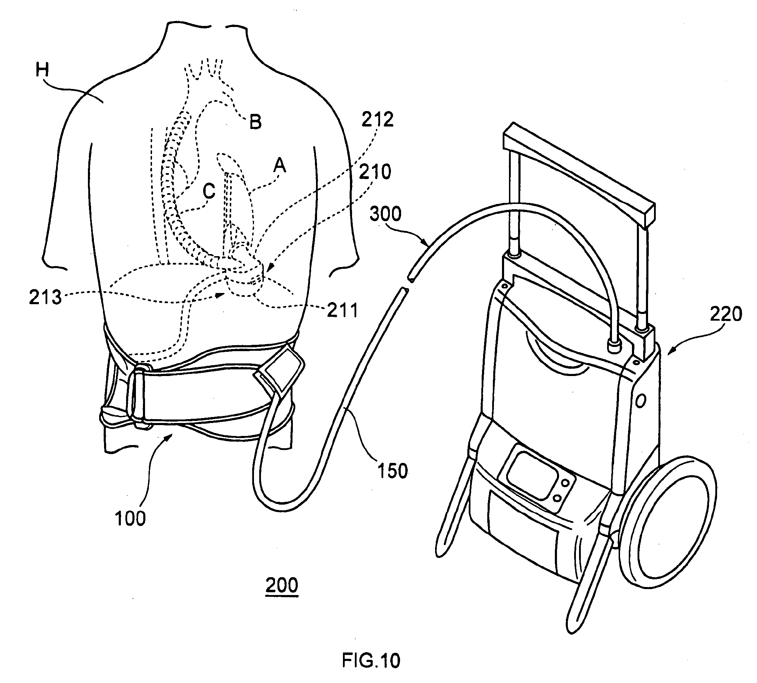

Next, a blood pump system that uses the skin-penetrating medical object holder 100 according to an embodiment of the present invention will be described using FIG. 10. FIG. 10 is a perspective view useful in explaining a blood pump system that uses the skin-penetrating medical object holder 100 according to the present embodiment.

As shown in FIG. 10, a blood pump system 200 includes a blood pump 210 that is implanted in a living body, an external controller 220 for driving and controlling the blood pump 210, the medical skin-penetrating object 150 that is used to connect the blood pump 210 and the external controller 220, and the skin-penetrating medical object holder 100 for holding the medical skin-penetrating object 150.

The blood pump 210 includes a pump driving unit 211 and a pump unit 212 and is constructed so that blood in the left ventricle A of the heart of the human body H flows into a pump casing 213 from an intake of the pump casing 213, is energized with flow energy by pump vanes (not shown) inside the pump casing 213, and then flows through an outlet of the pump casing 213 and an artificial blood vessel C implanted in the human body to the aorta B. It should be noted that a centrifugal pump, an axial-flow pump, a mixed-flow pump, or the like can be favorably used as the blood pump 210.

The external controller 220 includes a circulating liquid pump unit that supplies a circulating liquid to a periphery of a mechanical seal of the blood pump 210, a pump control unit that drives and controls the circulating liquid pump unit and the blood pump 210, a display unit that displays the operating states of the various components, a communication unit that exchanges information with external devices, a power supplying unit that supplies these components with electrical power, and a system control unit for controlling these components (none of such components are shown).

The medical skin-penetrating object 150 includes an inner tube for circulating liquid such as pure water between the blood pump 210 and the external controller 220, a cable that electrically connects the blood pump 210 and the external controller 220, and an outer tube that encloses the cable and the inner tube (none of such components are shown). By doing so, it is possible to safely and smoothly carry out treatment using the medical skin-penetrating object 150 after an operation that implants the blood pump 210.

Accordingly, since the blood pump system 200 of the present embodiment uses the skin-penetrating medical object holder 100 that can prevent the occurrence of infections from the outset, the blood pump system 200 is suited to treatments carried out after an operation that implants the blood pump 210.

It should be noted that although a case where the medical skin-penetrating object 150 is a medical skin-penetrating object used to connect the blood pump 210 implanted in the human body H and the external controller 220 used for driving and controlling the blood pump 210 has been described in the present embodiment, the present invention is not limited to this, and the medical skin-penetrating object 150 may be applied to a medical skin-penetrating object for circulating blood between a living body and a dialysis machine. In this case, it is possible to carry out dialysis treatment more safely and smoothly.

The medical skin-penetrating object for the present invention can also be applied in the same way to a medical skin-penetrating object used for carrying out PD. In this case, it is possible to carry out PD treatment more safely and smoothly.

In addition, the medical skin-penetrating object for the present invention can also be applied to a medical skin-penetrating object used in a drug delivery system. By doing so, it becomes possible to carry out treatment with a drug delivery system more safely and smoothly.

### Alternative Usage 1

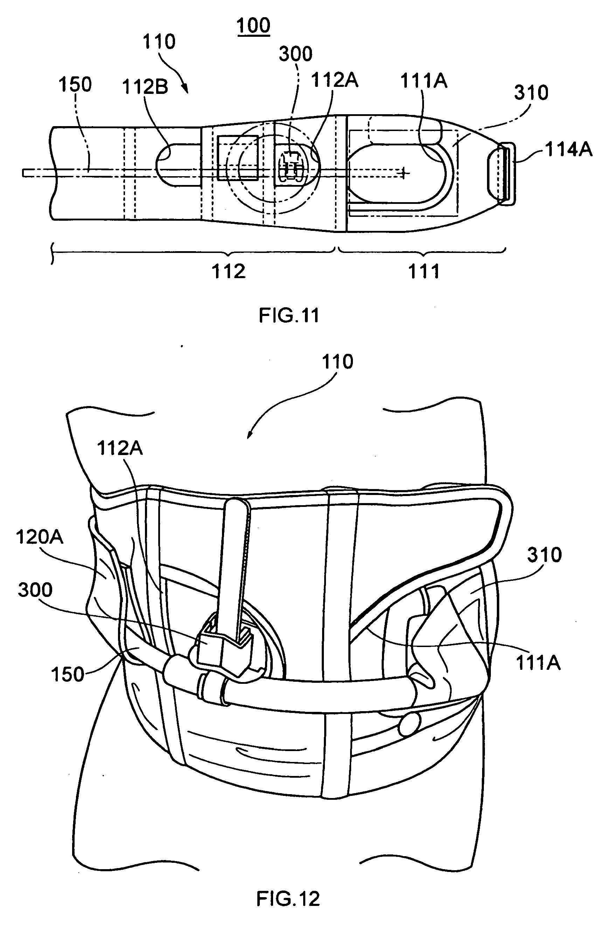

Although the case where the medical skin-penetrating object 150 is held by the penetrating object holding means 120 has been described in the above embodiment, the present invention is not limited to this and a supplementary holder 300 that is stuck to the living body may be exposed to the outside from the opening 112A of the holder part 112 shown in FIGS. 11 and 12, with the medical skin-penetrating object 150 being held by the supplementary holder 300 and the penetrating object holding parts 120A to 120C (only the penetrating object holding part 120A is shown).

By doing so, a state where the medical skin-penetrating object 150 is stably held on the living body is realized. In this case, the supplementary holder 300 is pressed onto the living body by the periphery of the opening 112A (the holder main body 110), so that the supplementary holder 300 is firmly held on the living body and the medical skin-penetrating object 150 can be held even more stably on the living body. This means that detachment of the medical skin-penetrating object 150 and the living body tissue due to external forces can be effectively suppressed, so that infections can be effectively prevented from occurring. Also, it is possible to apply medication to the living body (specifically, to the entry point of the medical skin-penetrating object 150 and a periphery thereof) from the penetrating object passage hole 111A and to provide a gauze 310 as a covering, so that infections can be prevented from occurring even more effectively.

### Alternative Usage 2

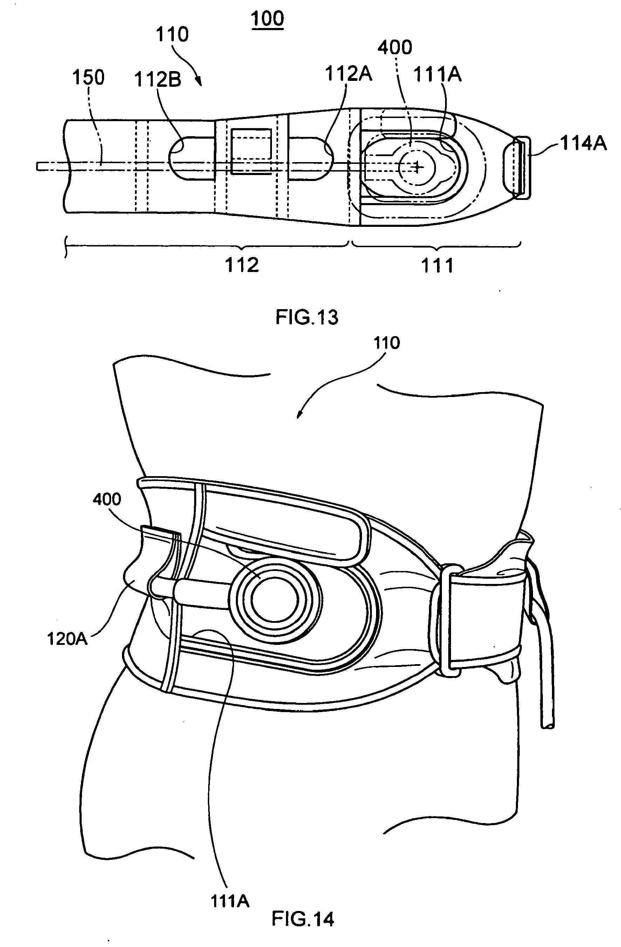

Also, for the present invention, as shown in FIGS. 13 and 14, a medical assembly 400 for sticking onto the living body may be exposed to the outside from the penetrating object passage hole 111A of the holder part 111, with the medical skin-penetrating object 150 being held by the medical assembly 400 and the penetrating object holding parts 120A to 120C (only the penetrating object holding part 120A is shown).

By doing so, a state where the medical skin-penetrating object 150 is stably held on the living body is realized. In this case, the supplementary holder 300 is pressed onto the living body by the edge of the opening 112A and the medical assembly 400 is pressed on the living body by the edge of the penetrating object passage hole 111A, so that the supplementary holder 300 and the medical assembly 400 are strongly held on the living body and a state where the medical skin-penetrating object 150 is held on the living body even more stably is realized. For this reason, detachment of the medical skin-penetrating object 150 and the living body tissue due to external forces can be suppressed more effectively, and infections can be prevented even more effectively.

A skin-penetrating medical object holder can effectively suppress detachment of a medical skin-penetrating object and living body tissue due to external forces, and therefore can prevent infections from occurring from the outset. The skin-penetrating medical object holder includes a belt-like holder main body that is wound around a living body and a penetrating object holding part that is disposed on the holder main body and holds a medical skin-penetrating object that passes through the skin of the living body. The penetrating object holding part is constructed so as to guide, in a state where the holder main body has been wound around the living body, the medical skin-penetrating object along a length direction of the holder main body.

## Claims

1. A skin-penetrating medical object holder (100) comprising:
a belt-like holder main body (110) that can be wound around a living body; and
a penetrating object holding means (120) that is disposed on the holder main body and holds a medical skin-penetrating object that passes through the skin of the living body,
wherein the holder main body has a penetrating object passage hole (111A) through. which the medical skin-penetrating object passes and a penetrating object guiding path (111B) that guides the medical skin-penetrating object in the penetrating object passage hole, and
an edge member (116) that opens and closes the penetrating object guiding path is attached to an edge of the penetrating object passage hole so as to be capable of separation and combining.

2. A skin-penetrating medical object holder according Claim 1,
wherein the penetrating object passage hole (111A) is a hole for carrying out a medical treatment to an entry point where the medical skin-penetrating object passes through the skin of the living body and a periphery thereof.

3. A skin-penetrating medical object holder according to Claim 1 or Claim 2,
wherein a wire-like member for reinforcing the skin-penetrating medical object holder is disposed at part of an edge of the penetrating object passage hole.

4. A skin-penetrating medical object holder according to Claim 3,
wherein an end part of the wire-like member is composed of a flexible member.

5. A skin-penetrating medical object holder according to any of Claim 1 to Claim 4,
wherein the penetrating object holding means has a plurality of penetrating object holding parts that are disposed at predetermined intervals along the length direction of the holder main body.

6. A skin-penetrating medical object holder according to any of Claim 1 to Claim 5,
wherein the penetrating object holding means includes two attaching parts (120A,120B) that can be detachably attached to one another and a penetrating object contacting part that is opened up and folded back via the attaching parts, and the penetrating object contacting part is constructed so that in a state where the two attaching parts are attached, the penetrating object contacting part is formed in a tube-like shape through which the medical skin-penetrating object is capable of passing, and
wherein the penetrating object contacting part is subjected to a treatment to stop the medical skin-penetrating object from slipping.

7. A skin-penetrating medical object holder according to any of Claim 1 to Claim 6,
wherein an attaching part for attaching a supplementary holder, which is stuck onto the living body and holds the medical skin-penetrating object, is disposed on the holder main body.

8. A skin-penetrating medical object holder according to Claim 7,
wherein in addition to the attaching part, an extra attaching part is disposed on the holder main body a predetermined distance from the attaching part along the length direction of the holder main body.

9. A skin-penetrating medical object holder according to any of Claim 1 to Claim 8,
wherein a plurality of wire-like members, which are oriented in a width direction of the holder main body, for achieving tight contact between the living body and the skin-penetrating medical object holder are disposed in a row at predetermined distances in the length direction.

10. A skin-penetrating medical object holder according to any of Claim 1 to Claim 9,
wherein the holder main body is formed of a woven material.

11. A skin-penetrating medical object holder according to Claim 10,
wherein the woven material is composed of fibers including a material that absorbs and radiates far-infrared radiation.

12. A skin-penetrating medical object holder according to Claim 10 or Claim 11,
wherein the holder main body is subjected to a stitching process so that a holder thickness at outer edge parts thereof is thicker than a holder thickness at a central part thereof.

13. A skin-penetrating medical object holder according to any of Claim 1 to Claim 12,
wherein the medical skin-penetrating object is used to connect a blood pump implanted inside the living body and an external controller for driving and controlling the blood pump.

14. A skin-penetrating medical object holder according to any of Claim 1 to Claim 12,
wherein the medical skin-penetrating object is used for circulating blood between the living body and a dialysis apparatus.

15. A skin-penetrating medical object holder according to any of Claim 1 to Claim 12,
wherein the medical skin-penetrating object is used to carry out peritoneal dialysis.

16. A skin-penetrating medical object holder according to any of Claim 1 to Claim 12,
wherein the medical skin-penetrating object is used in a drug delivery system.

17. A blood pump system (200) comprising:
a blood pump (210) for being implanted inside a living body;
an external controller (220) for driving and controlling the blood pump;
a medical skin-penetrating object (150) for connecting the blood pump and the external controller; and
a skin-penetrating medical object holder (110) for holding the medical skin-penetrating object,
wherein the skin-penetrating medical object holder is a skin-penetrating medical object holder according to any of Claim 1 to Claim 12.

## Patentansprüche

1. Halter (100) eines die Haut durchdringenden medizinischen Objektes mit:
einem riemenartigen Halterhauptkörper (110), der um einen lebenden Körper herumgewickelt werden kann; und
einer Durchdringungsobjekt-Halteeinrichtung (120), die an dem Halterhauptkörper angeordnet ist und ein die Haut durchdringendes medizinisches Objekt hält, das durch die Haut des lebenden Körpers tritt,
wobei der Halterhauptkörper ein Durchdringungsobjekt-Durchtrittsloch (111A), durch das das die Haut durchdringende medizinische Objekt tritt, und einen Durchdringungsobjekt-Führungspfad (111B) aufweist, der das die Haut durchdringende medizinische Objekt in dem Durchdringungsobjekt-Durchtrittsloch führt, und
einem Randelement (116), das den Durchdringungsobjekt-Führungspfad öffnet und schließt, an einem Rand des Durchdringungsobjekt-Durchtrittslochs so angebracht ist, dass es zu einem Separieren und Kombinieren in der Lage ist.

2. Halter eines die Haut durchdringenden medizinischen Objektes gemäß Anspruch 1,
wobei das Durchdringungsobjekt-Durchtrittsloch (111A) ein Loch ist zum Ausführen einer medizinischen Behandlung an einem Eintrittspunkt, an dem das die Haut durchdringende medizinische Objekt durch die Haut des lebenden Körpers tritt, und einer Umgebung von diesem.

3. Halter eines die Haut durchdringenden medizinischen Objektes gemäß Anspruch 1 oder 2,
wobei ein drahtartiges Element zum Verstärken des Halters des die Haut durchdringenden medizinischen Objektes an einem Teil eines Randes des Durchdringungsobjekt-Durchtrittslochs angeordnet ist.

4. Halter eines die Haut durchdringenden medizinischen Objektes gemäß Anspruch 3,
wobei ein Endteil des drahtartigen Elements aus einem flexiblen Element besteht.

5. Halter eines die Haut durchdringenden medizinischen Objektes gemäß einem der Ansprüche 1 bis 4,
wobei die Durchdringungsobjekt-Halteeinrichtung eine Vielzahl an Durchdringungsobjekt-Halteteilen hat, die unter vorbestimmten Intervallen entlang der Längsrichtung des Halterhauptkörpers angeordnet sind.

6. Halter eines die Haut durchdringenden medizinischen Objektes gemäß einem der Ansprüche 1 bis 5,
wobei die Durchdringungsobjekt-Halteeinrichtung zwei Befestigungsteile (120A, 120B), die lösbar aneinander angebracht werden können, und ein Durchdringungsobjekt-Kontaktierteil aufweist, das über die Befestigungsteile geöffnet und zurückgeklappt wird, und wobei das Durchdringungsobjekt-Kontaktierteil so aufgebaut ist, dass in einem Zustand, in dem die beiden Befestigungsteile befestigt sind, das Durchdringungsobjekt-Kontaktierteil in einer röhrenartigen Form ausgebildet ist, durch die das die Haut durchdringende medizinische Objekt zu einem Hindurchtreten in der Lage ist, und
wobei das Durchdringungsobjekt-Kontaktierteil einer Behandlung zum Beenden eines Rutschens des die Haut durchdringenden medizinischen Objektes unterworfen ist.

7. Halter eines die Haut durchdringenden medizinischen Objektes gemäß einem der Ansprüche 1 bis 6,
wobei ein Befestigungsteil zum Befestigen eines Hilfshalters, der an dem lebenden Körper anhaftet und das die Haut durchdringende medizinische Objekt hält, an dem Halterhauptkörper angeordnet ist.

8. Halter eines die Haut durchdringenden medizinischen Objektes gemäß Anspruch 7,
wobei zusätzlich zu dem Befestigungsteil ein Zusatzbefestigungsteil an dem Halterhauptkörper angeordnet ist und zwar in einem vorbestimmten Abstand von dem Befestigungsteil entlang der Längsrichtung des Halterhauptkörpers.

9. Halter eines die Haut durchdringenden medizinischen Objektes gemäß einem der Ansprüche 1 bis 8,
wobei eine Vielzahl an drahtartigen Elementen, die in einer Breitenrichtung des Halterhauptkörpers ausgerichtet sind, für das Erzielen eines dichten Kontaktes zwischen dem lebenden Körper und dem Halter des die Haut durchdringenden medizinischen Objektes in einer Reihe unter vorbestimmten Abständen in der Längsrichtung angeordnet sind.

10. Halter eines die Haut durchdringenden medizinischen Objektes gemäß einem der Ansprüche 1 bis 9,
wobei der Halterhauptkörper aus einem gewebten Material ausgebildet ist.

11. Halter eines die Haut durchdringenden medizinischen Objektes gemäß Anspruch 10,
wobei das gewebte Material aus Fasern besteht, die ein Material aufweisen, das fern-infrarote Strahlung absorbiert und ausstrahlt.

12. Halter eines die Haut durchdringenden medizinischen Objektes gemäß Anspruch 10 oder 11,
wobei der Halterhauptkörper einem Nähprozess so ausgesetzt ist, dass eine Halterdicke an seinen Außenrandteilen dicker als eine Halterdicke an seinem mittleren Teil ist.

13. Halter eines die Haut durchdringenden medizinischen Objektes gemäß einem der Ansprüche 1 bis 12,
wobei das die Haut durchdringende medizinische Objekt verwendet wird, um eine Blutpumpe, die im Inneren des lebenden Körpers implantiert ist, und eine externe Steuereinrichtung zum Antreiben und Steuern der Blutpumpe zu verbinden.

14. Halter eines die Haut durchdringenden medizinischen Objektes gemäß einem der Ansprüche 1 bis 12,
wobei das die Haut durchdringende medizinische Objekt verwendet wird für ein Zirkulieren von Blut zwischen dem lebenden Körper und einer Dialysevorrichtung.

15. Halter eines die Haut durchdringenden medizinischen Objektes gemäß einem der Ansprüche 1 bis 12,
wobei das die Haut durchdringende medizinische Objekt zum Ausführen einer Peritonealdialyse verwendet wird.

16. Halter eines die Haut durchdringenden medizinischen Objektes gemäß einem der Ansprüche 1 bis 12,
wobei das die Haut durchdringende medizinische Objekt in einem Medikamentspendesystem angewendet ist.

17. Blutpumpensystem (200) mit:
einer Blutpumpe (210), die dazu vorgesehen ist, im Inneren eines lebenden Körpers implantiert zu werden;
einer externen Steuereinrichtung (220) zum Antreiben und Steuern der Blutpumpe;
einem die Haut durchdringenden medizinischen Objekt (150) zum Verbinden der Blutpumpe und der externen Steuereinrichtung; und
einem Halter (110) des die Haut durchdringenden medizinischen Objektes zum Halten des die Haut durchdringenden medizinischen Objektes,
wobei der Halter des die Haut durchdringenden medizinischen Objektes ein Halter eines die Haut durchdringenden medizinischen Objektes gemäß einem der Ansprüche 1 bis 12 ist.

## Revendications

1. Support (100) pour un objet médical pénétrant dans la peau comprenant :
un corps principal (110) du support en forme de ceinture qui peut être enroulé autour d'un corps vivant ; et
un moyen de support (120) d'objet pénétrant qui est disposé sur le corps principal de support et qui supporte un objet médical pénétrant dans la peau qui passe à travers la peau du corps vivant,
où le corps principal de support dispose d'un trou de passage (111A) pour l'objet pénétrant à travers lequel l'objet médical pénétrant dans la peau passe et un chemin de guidage (111B) de l'objet pénétrant qui guide l'objet médical pénétrant dans la peau dans le trou de passage pour l'objet pénétrant,
et
un élément de bord (116) qui ouvre et ferme le chemin de guidage de l'objet pénétrant est fixé à un bord du trou de passage de l'objet pénétrant de façon à pouvoir séparer et combiner.

2. Support pour un objet médical pénétrant dans la peau selon la revendication 1,
où le trou de passage (111A) de l'objet pénétrant est un trou destiné à réaliser un traitement médical jusqu'à un point d'entrée où l'objet médical pénétrant dans la peau passe à travers la peau du corps vivant et sa périphérie.

3. Support pour un objet médical pénétrant dans la peau selon la revendication 1 ou 2,
dans lequel un élément en forme de fil pour renforcer le support pour l'objet médical pénétrant dans la peau est disposé au niveau d'une partie d'un bord du trou de passage de l'objet pénétrant.

4. Support pour un objet médical pénétrant dans la peau selon la revendication 3,
où une partie d'extrémité d'un élément en forme de fil est composée d'un élément flexible.

5. Support pour un objet médical pénétrant dans la peau selon l'une quelconque des revendications 1 à 4,
où le moyen de support de l'objet pénétrant possède une pluralité de parties de support de l'objet pénétrant qui sont disposées à intervalles prédéterminés le long de la direction de la longueur du corps principal de support.

6. Support pour un objet médical pénétrant dans la peau selon l'une quelconque des revendications 1 à 5,
où le moyen de support de l'objet pénétrant comporte deux parties de fixation (120A, 120B) qui peuvent être fixées l'une à l'autre de manière amovible et une partie en contact avec l'objet pénétrant qui s'ouvre et se rabat au moyen des parties de fixation, et la partie en contact avec l'objet pénétrant est formée de sorte que dans le cas où les deux parties de fixation sont attachées, la partie en contact avec l'objet pénétrant est formée en une forme tubulaire à travers laquelle l'objet médical pénétrant dans la peau peut passer, et
où la partie en contact avec l'objet pénétrant est soumise à un traitement pour empêcher l'objet médical pénétrant dans la peau de glisser.

7. Support pour un objet médical pénétrant dans la peau selon l'une des revendications 1 à la revendication 6,
où une partie de fixation pour attacher un support supplémentaire, qui est collé sur le corps vivant et qui maintient l'objet médical pénétrant dans la peau, est disposée sur le corps principal de support.

8. Support pour un objet médical pénétrant dans la peau selon la revendication 7,
où, en plus de la partie de fixation, une partie supplémentaire de fixation est disposée sur le corps principal de support à une distance prédéterminée de la partie de fixation le long de la direction de la longueur du corps principal de support.

9. Support pour un objet médical pénétrant dans la peau selon l'une quelconque des revendications de 1 à 8,
où une pluralité d'éléments en forme de fil, qui sont orientés dans une direction de largeur du corps principal de support, pour établir un contact étroit entre le corps vivant et le support de l'objet médical pénétrant dans la peau, sont disposés en une rangée à des distances prédéterminées dans la direction de la longueur.

10. Support pour un objet médical pénétrant dans la peau selon l'une quelconque des revendications de 1 à 9,
où le corps principal de support est constitué d'un matériau tissé.

11. Support pour un objet médical pénétrant dans la peau selon la revendication 10,
où le matériau tissé est composé de fibres formées d'un matériau qui absorbe et émet un rayonnement infrarouge lointain.

12. Support pour un objet médical pénétrant dans la peau selon la revendication 10 ou 11,
dans lequel le corps principal de support est soumis à un processus d'assemblage de sorte qu'une épaisseur de l'élément de support au niveau de ses parties de bords extérieurs soit plus grande qu'une épaisseur de l'élément de support au niveau de sa partie centrale.

13. Support pour un objet médical pénétrant dans la peau selon l'une quelconque des revendications de 1 à 12,
où l'objet médical pénétrant dans la peau est utilisé pour relier une pompe sanguine implantée à l'intérieur du corps vivant et une unité de commande externe pour entraîner et commander la pompe sanguine.

14. Support pour un objet médical pénétrant dans la peau selon l'une quelconque des revendications de 1 à 12,
dans lequel l'objet médical pénétrant dans la peau est utilisé pour faire circuler le sang entre le corps vivant et un appareil de dialyse.

15. Support pour un objet médical pénétrant dans la peau selon l'une quelconque des revendications de 1 à 12,
où l'objet médical pénétrant dans la peau est utilisé pour effectuer une dialyse péritonéale.

16. Support pour un objet médical pénétrant dans la peau selon l'une quelconque des revendications de 1 à 12,
où l'objet médical pénétrant dans la peau est utilisé dans un système d'administration de médicaments.

17. Système de pompe sanguine (20) comprenant :
une pompe sanguine (210) devant être implantée dans un corps vivant ;
une unité de commande externe (220) pour entraîner et commander la pompe sanguine ;
un objet médical (150) pénétrant dans la peau pour relier la pompe sanguine et l'unité de commande externe ; et
un support (110) pour l'objet médical pénétrant dans la peau pour soutenir l'objet médical pénétrant dans la peau,
où le support de l'objet médical pénétrant dans la peau est un support pour un objet médical pénétrant dans la peau selon l'une quelconque des revendications de 1 à 12.
